# EUROPEAN PATENT APPLICATION

(11) **EP 2 002 799 A1**
(43) Date of publication of application: **17.12.2008**
(21) Application number: 07011683.5
(22) Date of filing: 14.06.2007
(51) Int. Cl.: A61C 8/00, A61L 27/32

(54) **Ceramic medical implant having an osseconductive coating**

(71) Applicant: Nobel Biocare Services AG, 8058 Zürich-Flughafen (CH)
(72) Inventor: Hall, Jan, 416 80 Göteborg (SE); Jörneus, Lars, 430 30 Frillesas (SE); Wigren, Stina, 411 31 Göteborg (SE)
(74) Representative: Byström, Kurt Linus

(57) **Abstract**

A ceramic medical implant devised for permanent anchorage in bone tissue is disclosed. The ceramic medical implant has a coating on an outer porous surface thereon, wherein the outer porous surface has a surface roughness. The coating comprises calcium phosphate, applied with a coating thickness on the outer porous surface of the ceramic medical implant. A ratio of the coating thickness to the surface roughness is in a range of 0,5% to 30%, whereby osseointegration of the ceramic medical implant is promoted upon implantation of the ceramic medical implant. Moreover a method of producing such a ceramic medical implant is disclosed. According to an embodiment the ceramic medical implant is a ceramic dental implant.

## Description

### Field of the Invention

This invention pertains in general to the field of osseoconductive coatings on medical implants, and in particular to calcium phosphate coatings on medical implants, such as ceramic dental implants having a rough outer surface promoting osseointegration of the ceramic dental implants.

### Background of the Invention

Conventionally dental implants for anchoring dental restorations in bone tissue of a patient are made of titanium. Zirconia, zirconium oxide, is a material that is used for making dental restorations and it is desired to even produce dental implants of zirconia, amongst others of aesthetic reasons. However, it is difficult to provide a medical implant, such as a dental implant, i.e. an anchoring part of a dental restoration, in this material. This is due to the fact that dental implants have high requirements concerning mechanical strength of the dental implant, amongst others, which can only be fulfilled with highly dense ceramics, e.g. of sintered zirconia, alumina, or mixes thereof. However, highly dense sintered ceramics, e.g. of zirconia, alumina, or mixes thereof, have an outer surface structure, which usually is very smooth. The smooth outer surface is thus hard and provides excellent mechanical strength. However, a sintered ceramic dental implant, e.g. of zirconia, alumina, or mixtures thereof, that has such a hard, smooth outer surface involves inferior osseointegration of the medical implant with the bone tissue. It is known that smooth surfaces will not osseointegrates as well as moderately rough surfaces. Hence, a ceramic medical implant of this type is not suited for bearing dental restorations due to low stability of the medical implant in the bone tissue of the patient. That means that such a ceramic dental implant, which certainly has a high mechanical strength itself, nevertheless does not provide a stable basis for a dental restoration, once implanted in the bone tissue of the patient.

Thus, there is a need for an improved ceramic medical implant having improved osseointegrating properties of the medical implant upon implantation thereof.

### Summary of the Invention

Accordingly, embodiments of the present invention preferably seek to mitigate, alleviate or eliminate one or more deficiencies, disadvantages or issues in the art, such as the above-identified, singly or in any combination by providing a ceramic medical implant, a coating on an outer surface of such a medical implant, and a method of producing such a coating, according to the appended patent claims.

According to one aspect of the invention, a ceramic medical implant devised for permanent anchorage in bone tissue is provided. The ceramic medical implant comprises an osseoconductive coating on an outer porous surface thereon, wherein the outer porous surface of the ceramic medical implant having a surface roughness, wherein the osseoconductive coating comprises calcium phosphate and has a coating thickness on the outer porous surface of the ceramic medical implant, and wherein a ratio of a value of the coating thickness to a value of the surface roughness is in a range of 0,5% to 30%, whereby osseointegration of the ceramic medical implant is promoted upon implantation of the ceramic medical implant.

According to another aspect of the invention, a method of producing an osseoconductive coating on an outer porous surface of a ceramic medical implant according to the first aspect of the invention is provided. The method comprises providing the outer porous surface of the ceramic medical implant, the outer porous surface having a surface roughness, and applying an osseoconductive coating comprising calcium phosphate with a coating thickness on the outer porous surface of the ceramic medical implant, and with a ratio of a value of the coating thickness to a value of the surface roughness in a range of 0,5% to 30%, whereby osseointegration of the ceramic medical implant is promoted upon implantation of the ceramic medical implant.

In an embodiment, the range is approximately 1% to 10%.

Further embodiments of the invention are defined in the dependent claims.

Some embodiments of the invention provide for improved osseointegration of a ceramic medical implant.

Some embodiments of the invention provide for accelerated secondary stability of a ceramic medical implant.

Some embodiments of the invention provide for improved ceramic dental implants.

Some embodiments of the invention provide for improved osseointegration by means of a calcium phosphate coating on an outer porous surface structure of a ceramic dental implant. The medical implant may have a surface roughness, wherein the calcium phosphate coating does not substantially alter a geometrical topography of the outer porous surface structure.

A thickness of the calcium phosphate coating may be relatively thin compared to the geometrical topography of the outer porous surface, but the calcium phosphate coating still completely covers the outer porous surface and penetrates into the porous structure thereof. In this manner a sufficient amount of calcium phosphate for providing improved osseointegrating properties of the outer porous surface structure of the ceramic medical implant is presented.

Some embodiments provide for a calcium phosphate coating that has a coating thickness, which provides a coating on a ceramic medical implant, which completely covers a porous surface thereof.

On the other hand the thickness of the calcium phosphate coating may not be too thick as too thick coating form cracks and delaminate. Too thick coatings also may affect the coloring of the ceramic medical implant.

Some embodiments provide for a calcium phosphate coating on a ceramic medical implant that has a coating thickness, which is less susceptible to crack formation than some previously known coatings.

Still, some embodiments of the invention provide for a calcium phosphate coating on a ceramic medical implant, which coating is so thin that particle release from the coating does not occur.

Some embodiments of the invention provide for a calcium phosphate coating on an outer porous surface structure of a ceramic medical implant having a surface roughness, wherein the calcium phosphate coating does not substantially alter a color of the ceramic medical implant and the aesthetic appearance thereof is preserved.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which embodiments of the invention are capable of will be apparent and elucidated from the following description of embodiments of the present invention, reference being made to the accompanying drawings, in which
Fig. 1 is a Surface Electron Microscope (SEM) picture taken perpendicular to the surface of a zirconia medical implant before surface roughening thereof, wherein the width of the picture is 100µm;
Fig. 2 is a SEM picture taken perpendicular to the surface of the zirconia medical implant of Fig. 1 after surface roughening, wherein the width of the picture is 100µm;
Fig. 3 is a schematic illustration of a ceramic medical implant implanted in a bone tissue;
Fig. 4 is a schematic illustration of a macro roughness in form of a threaded part of the ceramic medical implant shown in Fig. 3;
Fig. 5 is a schematic illustration of a coating applied on an outer surface of the ceramic medical implant of Fig. 3, having a micro roughness to promote osseointegration;
Fig. 6 is a schematic illustration of a coating of calcium phosphate applied the outer surface shown in Fig. 5, providing a coating on the outer surface of the medical implant of Fig. 3; and
Fig. 7 is a flow chart illustrating an embodiment of a method of producing a ceramic medical implant having a coating applied thereon.

### Description of embodiments

Embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

According to embodiments, a coating on a ceramic medical implant, such as a medical implant for permanent anchoring in bone tissue, having an outer surface having a given geometry or topography is provided, which is advantageous for the total osseointegration of the medical implant upon implantation. Advantageously an improved osseoconductive effect is combined with an advantageous osseointegrating effect of the outer surface.

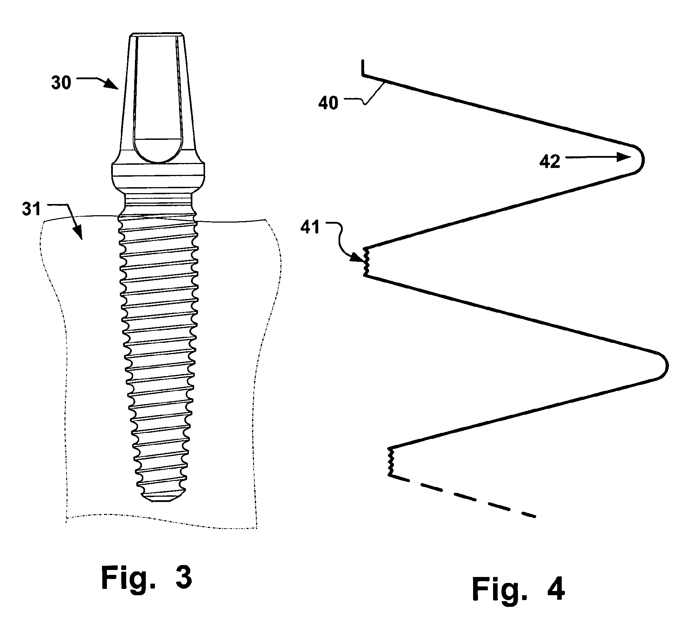

In Fig. 3 a schematic illustration of an embodiment of a ceramic medical implant 30 implanted in a bone tissue 31 is shown. The ceramic medical implant 30 comprises an anchoring part for anchoring within the bone tissue of the patient and comprises a mounting part for receiving a dental restoration element to be attached thereto, such as a crown, a bridge, or a prosthesis construction.

Medical implants according to embodiments have at least one outer surface devised for implantation in a bone tissue, such a jaw bone tissue or craniofacial bone tissue.

The outer surface, hereinafter also called "surface" for the sake of simplicity, may have a topography with a macro roughness, e.g. in the form of one or several threads in order to mechanically anchor the medical implant in the bone tissue.

Topography is amongst others defined as the study or detailed description of the surface features of a region of an object, here of the medical implant. The medical implant has an outer surface and the geometry thereof follows a defined topography both on a macro scale and a micro scale, as will now be described.

The macro roughness defines the outer geometry of the medical implant. Fig. 4 is a schematic illustration of a macro roughness. In Fig. 4 the macro roughness is illustrated in the form of a threaded part of the ceramic medical implant 30 shown in Fig. 3. Additionally, the macro roughness of the threaded outer surface 40 may in itself have a surface roughness, for instance defined as a porosity of the surface, but this is referred to below as micro roughness. The macro roughness of the surface lies for instance for a medical implant in the mm range, wherein the macro roughness may further be subdivided into finer structures, such as grooves 41 on threaded portions, as illustrated in Fig. 4, or a neck of the medical implant 30 in the sub mm range.

Furthermore, the outer surface of the medical implant may have a micro roughness in the µm range. The micro roughness defines the topography of the surface of medical implant in the present context. In contrast, the above-described macro roughness is defined by the outer contour of the medical implant, as for instance shown in Fig. 4.

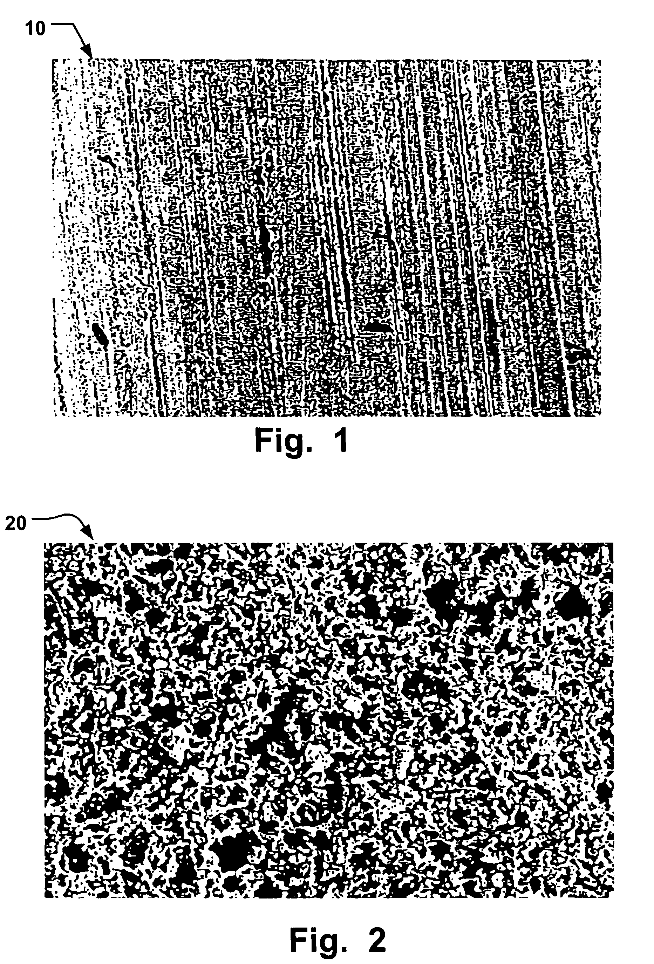

Some examples for different micro roughnesses are given in Fig. 1 and Fig. 2.

Fig. 1 shows an image of the outer surface topography of a machined and densely sintered ceramic medical implant without a processed outer surface thereof, i.e. after sintering the ceramic medical implant. The outer surface has a very fine micro roughness, i.e. a smooth outer surface that has disadvantageous properties with regard to osseointegration of the medical implant, as indicated above in the background section of this description.

A more coarse or rough outer surface is shown in the image of Fig. 2. The outer surface has an increased micro roughness compared to the outer surface shown in Fig. 1. Such a more coarse or rough outer surface of a medical implant improves osseointegration thereof.

Such a coarse or rough outer surface may for instance be provided in the form of a porous, rough surface layer, having an outer surface roughness, for instance with a Ra value (explained below) in the µm range.

For example, in WO 2005/027771, of the same applicant as the present application, which is incorporated herein by reference in its entirety, discloses a densely sintered ceramic medical implant having a ceramic layer arranged thereon. The ceramic layer, and thus an outer surface of the dental implant, is provided with a surface that has a porosity, which is larger or has more pores than in the underlying densely sintered ceramic material. In this manner, the disclosed ceramic medical implant fulfills the requirement of mechanical strength, and a considerable improvement of osseointegration is achieved. Thus, the ceramic layer provides a micro roughness of the ceramic medical implant that is advantageous for osseointegration of the ceramic medical implant in bone tissue, i.e. the ingrowth of bone tissue into the pores of the surface of the ceramic dental implant.

Alternatively to a rough porous surface layer on a sintered ceramic medical implant, the outer micro roughness of a ceramic medical implant may be provided on a ceramic substrate thereof by modifying the sintered outer surface, e.g. by chemical or mechanical abrasion methods, as for instance disclosed in US-A1-2005/0106534.

A porous surface, independent of its manufacturing method, has a micro roughness that may promote osseointegration. Ceramic dental implants having a suitable surface roughness reach immediately upon implantation a primary stability in bone tissue by the macro roughness, i.e. the macro structure of e.g. threads of the ceramic dental implant. The ceramic medical implant then osseointegrates with the surrounding bone tissue within a healing time of about 3 to 4 months, so that a secondary stability is provided, i.e. a permanent bond between the threaded anchoring part of the ceramic medical implant screwed into the bone tissue and the bone tissue is provided. However during a certain time following the implantation, the total stability of the medical implant in the bone tissue decreases to a certain stability that is lower than the initial stability, i.e. the stability of the medical implant shows a stability dip after implantation. Then bone growth accelerates and osseointegration is achieved. The secondary stability is provided by pores of the rough surface, into which the bone tissue is growing firmly.

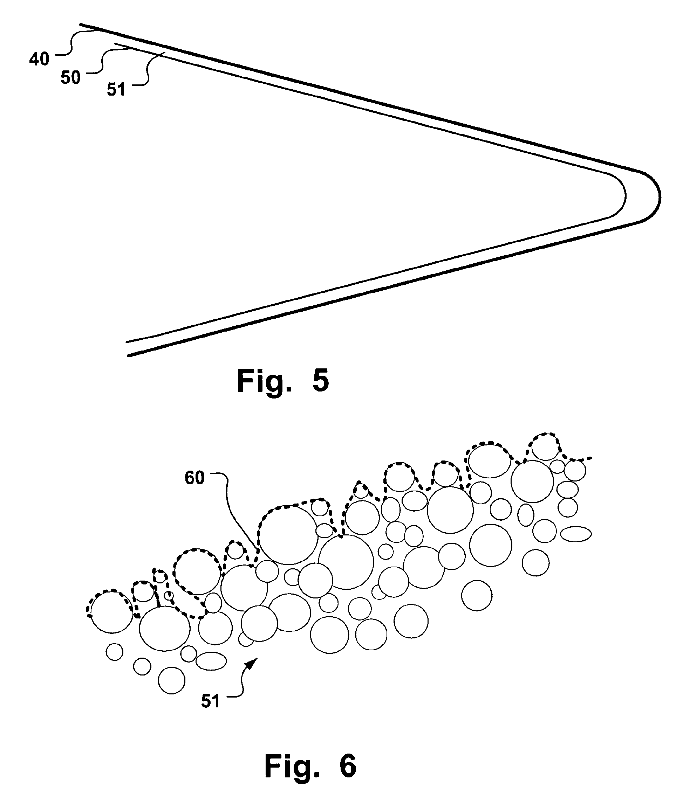

Fig. 5 is a schematic illustration of a coating 51 applied on the ceramic medical implant of Fig. 3, providing an outer surface 50 having a micro roughness to promote osseointegration.

Moreover, the outer surface 50 of the medical implant may have a coating. The coating may be provided as illustrated in Fig. 6, i.e. in the form of a coating 60, e.g. a nanocoating, on top of the surface 50 that itself has an elevated micro roughness. The coating 60 on the resulting surface may have a thickness in the nm range. The coating 60 substantially follows the topography of the porous rough outer surface 50.

In WO 2005/123579 of Promimic AB, a coating of calcium phosphate is disclosed. More precisely, in WO 2005/123589, a synthetic nano-sized crystalline calcium phosphate is disclosed forming a coating on a surface of a medical implant providing improved osseointegration. However, WO 2005/123579 fails to provide guidance concerning suitable thicknesses of such coatings with regard to an existing surface of a medical implant. In WO 2005/123579 only a maximum layer thickness of 150nm synthetic nano-sized crystalline calcium phosphate is mentioned. Calcium phosphate itself is osseoconductive, i.e. it may accelerate an existing bone ingrowth process.

When having a porous surface structure on a dental implant, such as disclosed in WO 2005/027771, or US-A1-2005/0106534, it is vital for the osseointegrating effect thereof that the topography is not geometrically influenced by an overlying osseoconductive layer. Only in this case the total osseointegrating effect of the micro roughness and the coating is improved. When a coating layer thereon is too thick, i.e. the topography of the micro roughness is influenced, osseointegration of the medical implant may be inferior compared to a similar implant without a coating.

Long-term bone stability itself is not improved by the coating thus provided. However, both the micro and macro roughness is essential for the long-term stability. Osseointegration is still determined by the micro roughness. In case the coating of the surface influences the micro roughness, this has proven to be disadvantageous.

This applies also to calcium phosphate coatings, i.e. in case a calcium phosphate coating on a ceramic medical implant is too thick, osseointegration of the ceramic medical implant is deteriorated in total. On the other hand, in case the calcium phosphate coating is too thin, no improvement of osseoconductivity is achieved compared to an uncoated medical implant because the mass of calcium phosphate provided is too little to give an osseoconductive effect. With other words, a coating of calcium phosphate that has such a large surface layer thickness that the micro roughness is substantially leveled out, i.e. made more smooth or even, certainly improves osseoconductivity of the dental implant, but considerably deteriorates the osseointegration of the dental implant. Consequently, in this case anchoring of the medical implant as a whole is deteriorated by the coating. Therefore, it is desired to provide a coating of a ceramic medical implant that does not substantially alter the effects and properties of a micro roughness of the same surface.

For instance when having a porous surface structure on a dental implant, such as disclosed in WO 2005/027771, it is vital for the osseointegrating effect thereof that the topography is not substantially geometrically influenced by an overlying osseoconductive layer, such as disclosed in WO 2005/123579. In WO 2005/123579 no hint is given that this condition is of importance for the total osseointegrating effect of the dental implant.

Fig. 6 is a schematic illustration of a coating of calcium phosphate applied to the outer surface shown in Fig. 5, providing a coating of the outer surface of the medical implant of Fig. 3. As can be seen in the illustration of Fig. 6, the coating 60 substantially follows the micro roughness of the ceramic porous surface layer 51, keeping substantially intact the osseointegrative properties of the ceramic porous surface layer 51. However, the coating 60 adds osseoconductive properties to the ceramic porous surface layer 51.

Applicants have found that osseointegration of ceramic dental implants is promoted advantageously upon implantation of the dental implants, when the implants are provided with a coating that comprises calcium phosphate, wherein the coating has a coating thickness value that may contribute to achieving improved osseointegration. More specifically, according to an embodiment improved osseointegration is provided by a coating thickness value that fulfills a ratio of 0,5% to 30% in relation to a surface roughness of the ceramic implant's outer surface, on which the coating that comprises calcium phosphate is applied on. Surface roughness in the context of ratios described herein refers to micro roughness as explained above.

A homogenous distribution of the coating that comprises calcium phosphate with regularly distributed layers is sometimes difficult to accomplish with certain geometries of the dental implants, e.g. in a threaded profile. Here, the value of an average thickness may contribute to achieving an advantageous range of 0,5% to 30%, whereby different distributions of the coating on the medical implant are considered, e.g. that a thicker coating is applied in the root of the thread.

Applicants have found that embodiments of the invention provide for an acceleration of reaching secondary stability, for instance already after 1-2 months. Furthermore embodiments provide for a reduction of the depth of the stability dip upon implantation. This means that the medical implants according to embodiments loose less stability than previously known upon implantation and reach secondary stability faster than previously possible. Implantation thus becomes more reliable. Still, primary stability is achieved that is not deteriorated, compared with previously known dental implants. Secondary stability is achieved faster than without a calcium phosphate coating in the advantageous range.

In ceramic dental implants, where the pores of the rough surface structure are clogged by a coating applied thereon, secondary stability may not be achievable.

According to an embodiment, a ratio of the average coating thickness of a coating, which comprises calcium phosphate on the ceramic medical implant surface, to the average surface roughness of the micro roughness of a ceramic medical implant surface is in a range of 0,5% to 30%.

Furthermore, coatings that comprise calcium phosphate that have a coating thickness value that may contribute to achieving improved osseointegration, provided in the above mentioned advantageous range of 0,5% to 30%, may not influence the color of the medical implant and the aesthetic appearance thereof is preserved.

Moreover, coatings that comprise calcium phosphate that have a coating thickness value that may contribute to achieving improved osseointegration, provided in the above mentioned advantageous range of 0,5% to 30%, may avoid acid attacks and deposits when the medical implant is implanted in bone tissue.

Still, coatings that comprises calcium phosphate that have a coating thickness value that may contribute to achieving improved osseointegration, provided in the above mentioned advantageous range of 0,5% to 30%, may have a sufficiently large thickness, such that the coating does not form cracks which may lead to a delamination of the coating.

For instance, a ceramic medical implant having a Ra roughness value, which is explained more detailed below, of 2-3 µm and has a coating thickness of 30-300 nm ensures that the surface having a desired roughness, does not get filled with coating material. That means the coating does not substantially alter the micro roughness of the ceramic dental implant.

One particular calcium phosphate is Hydroxyapatite, HA, Ca₁₀(PO₄)₆(OH)₂, which is one of the major mineral components in animal and human bodies, which for instance gives hardness and strength to bone and teeth. Hydroxyapatite may be comprised in the above described calcium phosphate coatings according to some embodiments.

According to some embodiments, dental implants having a porous surface, as for instance described in WO 2005/027771 or US-A1-2005/0106534, are coated with a coating comprising calcium phosphate. In more detail, a ceramic medical implant surface on at least a portion of the dental implant's total surface, is arranged for anchoring in a bone tissue in a patient. The ceramic medical implant surface is provided with a porous surface, i.e. the surface has a surface micro roughness. Further, the ceramic medical implant surface is provided with a coating that comprises calcium phosphate. The coating is applied on the porous surface with a coating thickness thereon. According to some embodiment, the coating thickness is constant over the porous surface. According to embodiments the coating thickness is substantially constant over the porous surface. According to some embodiments, the coating thickness may be non constant over the porous surface. In the two latter cases an average thickness of the coating on the porous surface may be measured.

A measure for surface roughness are parameters, such as Ra value (mean roughness), Rt value (maximum roughness), Sa value etc. The mean or average roughness (Ra) is defined as the average of absolute values of a number of measurements across a surface of interest. Sa is the equivalent amplitude parameter on a 3D or areal basis.

Roughness measurements may for instance be performed according to procedures as determined in international standards. However, measurements in accordance with such international ISO standards may prove difficult to implement on dental implants, e.g. due to the small surfaces thereof.

Attempts have been made to define and measure surface roughness of prior art dental abutment and dental implants (see e.g., Wennerberg, Ann et al., Design and Surface Characteristics of 13 Commercially Available Oral Implant Systems, JOMI, Vol. 8, No. 6 pages 622-633, (1993), which hereby is incorporated by reference in its entirety). The Wennerberg article defines two surface roughness parameters: (i) Rt, which is the maximum peak to valley height of the profile of the surface (see page 623) and (ii) Ra, which is the mean value of the peak to valley distance. Using the Wennerberg definitions, smooth machined and/or polished surface of prior art abutments and implants have a Rt of approximately 10 microns or less and a Ra of approximately 0.6 microns or less.

In an embodiment, the range is approximately 1% to 10%, such as 1% to 10%. The range may be approximately 2% to 10%, 3% to 8%, 3,5% to 7%, or 4% to 6%.

In an embodiment, the range is approximately 10% to 30%. Regulatory requirements in some countries necessitate that parameters of medical implants are measured or measurable, e.g. for certification purposes. However, very thin coatings may be difficult to measure. For instance coating thicknesses less than 100nm might be difficult to measure. Still, embodiments with coating thicknesses less than 100nm may be manufactured.

For instance, a ceramic medical implant may have a coating thickness of 100nm and surface roughness of 1 µm, which results in a ratio of 10%. Correspondingly, a coating thickness of 300nm and surface roughness of 1 µm results in a ratio of 30%. Thicker coatings, or at least substantially thicker coatings, might form cracks and delaminate.

A lower limit of the coating thickness and thus of the ratio may be determined by available manufacturing methods and processes. For instance, for a surface roughness of a medical implant of 1 µm, a minimum manufacturable coating thickness of 10 nm results in a ratio of 1%, etc.

Embodiments of a method of producing a coating on a medical implant will now be described. More precisely, different ways of applying a coating that comprises calcium phosphate and has a thickness value that fulfills the ratio of 0,5% to 30% in relation to the average surface roughness of the ceramic implant's surface on which the coating that comprises calcium phosphate is applied on, are described now.

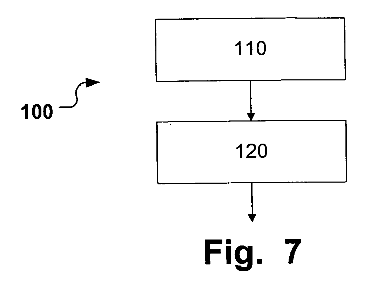

Fig. 7 is a flow chart illustrating an embodiment of a method 100 of producing a ceramic medical implant having a coating applied thereon. The method comprises providing, illustrated as step 110, an outer porous surface of a ceramic dental implant, wherein the outer porous surface has a surface micro roughness.

A number of alternative manufacturing processes for providing a ceramic porous surface, are for instance described in detail in WO 2005/027771. The manufacturing processes described therein have in common that they are adding a ceramic porous layer on a substrate, instead of removing material from the substrate in order to provide a porous surface. These manufacturing processes have a number of advantages in comparison with material removing processes. For instance material may be removed by etching with HF acid. HF acid is difficult to handle due to its aggressivity. Furthermore reliable, repeatable results of a desired surface roughness are difficult to achieve.

However, the desired surface roughness may be achieved by treating the surface of a ceramic substrate with other methods, e.g. by sand blasting, or by other subtractive methods as described in US-A1-2005/0106534.

As illustrated at step 120, a coating comprising calcium phosphate is applied to the outer porous surface with a coating thickness onto the ceramic dental implant. The coating is applied with a ratio of the surface micro roughness to the coating thickness in a range of 0,5% to 30%, whereby combined osseoconduction and osseointegration properties of the ceramic medical implant are improved.

For instance US 2002/0038149 A1 of the same applicant as the present application discloses methods of producing coatings that comprise calcium phosphate on medical implants for permanent anchorage in bone tissue. Although the methods described in US 2002/0038149 A1 are applied to implants made of a biocompatible material, such as titanium, applicants have found that these methods may likewise be applied to ceramic implants. US 2002/0038149 A1 is herewith incorporated by reference in its entirety, in particular the section describing coating methods in detail. The medical implants have at least one roughened surface intended to face the tissue in the implantation region, which is made of a biocompatible material, such as provided by a ceramic coating disclosed in WO 2005/027771. The medical implant is by a coating method provided with a thin, uniform and adherent calcium-phosphate coating with a well controlled dissolution rate. The coating follows the topography of the underlying roughened surface in order to combine properties for rapid bone growth (osseoconductive effect) during the healing phase and properties for a guaranteed long-term stability during clinical loading conditions (osseointegrating effect).

In more detail, methods of producing a coating that comprises calcium phosphate according to some embodiments, are for instance Plasma Spraying, Laser Ablation, Ion Beam Assisted Sputtering and Ion Beam Sputtering, Wet Chemical Coating Methods, RF Sputtering, Physical Vapor Deposition (PVD), Chemical Vapor Deposition (CVD), etc.

Application of HA by means of Wet Chemical Coating on a dental implant provides for avoiding shadow areas that are not coated, which might occur for instance when using PVD on a threaded dental implant. Thus a reliable, homogenous coating is ensured.

### Examples:

Dental implants were produced by the above mentioned method of production from a Metoxit ceramic raw material. Fig. 1 is a Surface Electron Microscope (SEM) picture taken perpendicular to an outer surface 10 of a zirconia medical implant before surface roughening thereof, wherein the width of the picture is 100µm. Fig. 2 is a SEM picture taken perpendicular to an outer porous surface 20 of a zirconia medical implant corresponding to Fig. 1, but after surface roughening thereof, wherein the width of the picture is 100µm.

Geometries of the examples were: diameter=4,3 mm and 5mm, length=10,13 and 16 mm. The porous modified surface comprising zirconia, which is a well known bio ceramic material (ISO 13356), was of yttrium stabilized tetragonal zirconia (Y-TZP). The porous modified surface 20 was produced by dropp coating.

Measurements made on the implemented ceramic medical implant having a threaded portion for attachment in bone tissue were:
Flank of thread at upper part of implant: Ra=1,44µm, Rt=16,20µm;
Flank of thread at lower part of implant: Ra=1,86µm, Rt=13,51µm;
Flank of thread at upper part of implant: Ra=0,5µm, Rt=12,73µm;
Flank of thread at lower part of implant: Ra=0.97µm, Rt=11,37µm.

Hence, an average Ra value for the implant was Ra_average=1,19µm, and an average Rt value for the implant was Rt_average=13,45µm.

Removal torque and bone implant contact were equal to or better than that of a comparable TiUnite^{®} surface of a medical implant made of titanium. Strength measurements were similarly good.

A calcium phosphate coating on this threaded implant surface is according to an embodiment applied with an average layer thickness, based on the measured Rt_average value in the range of 1,19nm to 238nm.

As used herein, the singular forms "a", "an" and "the" are intended to include the plural forms as well, unless expressly stated otherwise. It will be further understood that the terms "includes," "comprises," "including" and/or "comprising," when used in this specification, specify the presence of stated features, integers, steps, operations, elements, and/or components, but do not preclude the presence or addition of one or more other features, integers, steps, operations, elements, components, and/or groups thereof. Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

The present invention has been described above with reference to specific embodiments. However, other embodiments than the above described are equally possible within the scope of the invention. The different features and steps of the invention may be combined in other combinations than those described. The scope of the invention is only limited by the appended patent claims.

## Claims

1. A ceramic medical implant devised for permanent anchorage in bone tissue, said ceramic medical implant comprising an osseoconductive coating on an outer porous surface thereon, wherein
said outer porous surface of said ceramic medical implant having a surface roughness, wherein
said osseoconductive coating comprises calcium phosphate and has a coating thickness on said outer porous surface of said ceramic medical implant, and wherein
a ratio of a value of said coating thickness to a value of said surface roughness is in a range of 0,5% to 30%, whereby osseointegration of said ceramic medical implant is promoted upon implantation of said ceramic medical implant.

2. The ceramic medical implant according to claim 1, wherein said surface roughness is an average surface roughness (Ra) of said outer porous surface of said ceramic medical implant.

3. The ceramic medical implant according to claim 1 or 2, wherein said coating thickness is an average coating thickness of said coating on said outer porous surface of said ceramic medical implant.

4. The ceramic medical implant according to any preceding claim, wherein said range is approximately 1% to 10%.

5. The ceramic medical implant according to any of claims 1 to 3, wherein said range is approximately 10% to 30%.

6. The ceramic medical implant according to any preceding claim, wherein said outer porous surface is a ceramic surface of a material based on zirconia or alumina.

7. The ceramic medical implant according to claim 4 or 5, wherein said outer porous surface is roughened or micro structured.

8. The ceramic medical implant according to any preceding claim, wherein said outer surface of said ceramic medical implant is intended to face the bone tissue in an implantation region thereof and is at least partly provided with said coating.

9. The ceramic medical implant according to any preceding claim wherein said calcium phosphate is hydroxyapatite, HA, Ca₁₀(PO₄)₆(OH)₂.

10. The ceramic medical implant according to any preceding claim wherein said ceramic medical implant is a ceramic dental implant

11. A method of producing an osseoconductive coating on an outer porous surface of a ceramic medical implant according to any of claims 1 to 10, comprising
providing said outer porous surface of said ceramic medical implant, said outer porous surface having a surface roughness, and
applying an osseoconductive coating comprising calcium phosphate with a coating thickness on said outer porous surface of said ceramic medical implant, and with a ratio of a value of said coating thickness to a value of said surface roughness in a range of 0,5% to 30%, whereby osseointegration of said ceramic medical implant is promoted upon implantation of said ceramic medical implant.

12. The method according to claim 11, wherein said applying of said coating comprises applying said coating on said outer porous surface of said ceramic medical implant by physical vapor deposition or a wet chemical coating method.
